# EUROPEAN PATENT APPLICATION

(11) **EP 4 104 828 A1**
(43) Date of publication of application: **21.12.2022**
(21) Application number: 21754361.0
(22) Date of filing: 09.02.2021
(51) Int. Cl.: A61K 31/196, A61K 9/70, A61K 47/06, A61K 47/12, A61K 47/14, A61K 47/20, A61K 47/32, A61K 47/34, A61P 29/00

(54) **DICLOFENAC SODIUM-CONTAINING PATCH**

(30) Priority: 12.02.2020 JP 2020021284
(71) Applicant: Hisamitsu Pharmaceutical Co., Inc., Tosu-shi, Saga 841-0017 (JP)
(72) Inventor: OKAWA Koji, Tokyo 100-6330 (JP); OTAKA Kuniaki, Tokyo 100-6330 (JP); SATO Hiroyuki, Tokyo 100-6330 (JP); INAKURA Hiroshi, Tokyo 100-6330 (JP); KURIKI Masaaki, Tokyo 100-6330 (JP)
(74) Representative: Grünecker Patent- und Rechtsanwälte PartG mbB
(86) International application number: PCT/JP2021/004741
(87) International publication number: WO 2021/161985

(57) **Abstract**

Disclosed is a patch for alleviating cancerous pain. The patch is provided with a support layer and an adhesive layer laminated on the support layer. The adhesive layer contains an adhesive base and 150-225 mg of diclofenac sodium. One patch is used by being attached once per day.

## Description

### Technical Field

The present invention relates to a diclofenac sodium-containing patch.

### Background Art

Patent Literature 1 discloses a patch comprising a non-steroidal anti-inflammatory drug.

### Citation List

### Patent Literature

[Patent Literature 1] WO 2018/124089

### Summary of Invention

### Technical Problem

It is desirable to provide a patch that can sufficiently relieve cancer pain when administered to cancer patients. However, there are still no products that have been put on the market for diclofenac sodium-containing patches relieving cancer pain, and therefore the desired dosage and administration for the patches are not clear.

### Solution to Problem

In order to solve the above-described problem, the present inventors have conducted extensive studies. As a result, they have found that a sufficient effect of relieving cancer pain can be obtained by administering a patch comprising 150 mg to 225 mg of diclofenac sodium once a day, thus leading to realization of the present invention.

A patch for relieving cancer pain of the present invention is a patch comprising a backing layer and an adhesive layer laminated on the backing layer, wherein the adhesive layer comprises an adhesive base and 150 mg to 225 mg of diclofenac sodium, and the patch is used by applying the patch once a day.

### Advantageous Effects of Invention

The patch of the present invention exhibits a sufficient effect of relieving cancer pain.

### Brief Description of Drawings

FIG. 1 is a graph showing the Kaplan-Meier curve for an administration group of 75 mg of diclofenac sodium.
FIG. 2 is a graph showing the Kaplan-Meier curve for an administration group of 150 mg of diclofenac sodium.
FIG. 3 is a graph showing the Kaplan-Meier curve for an administration group of 225 mg of diclofenac sodium.
FIG. 4 is a graph showing the Kaplan-Meier curve for an administration group of 150 mg or 225 mg of diclofenac sodium.

### Description of Embodiments

The patch of the present invention comprises a backing layer and an adhesive layer laminated on the backing layer. The adhesive layer is usually laminated on one surface of the backing layer, and a peelable film is laminated on the other surface of the adhesive layer as necessary.

First, the adhesive layer will be described. The adhesive layer is a site to be pressed onto the skin during application of the patch and comprises an adhesive base and diclofenac sodium.

Diclofenac sodium is a non-steroidal anti-inflammatory drug and is a component for relieving cancer pain. Each patch comprises 150 mg to 225 mg of diclofenac sodium.

The adhesive base may be at least one adhesive base selected from a rubber adhesive base, an acrylic adhesive base, and a silicone adhesive base. The rubber adhesive base is, for example, polyisoprene, polyisobutylene (PIB), polybutadiene, a styrene-butadiene-styrene block copolymer, a styrene-isoprene-styrene (SIS) block copolymer, styrene-butadiene rubber, styrene-isoprene rubber, or a combination thereof. Among these, a SIS block copolymer, PIB, or a combination thereof is preferable and a mixture of an SIS block copolymer and PIB is more preferable from the viewpoint of enhancing skin permeability of diclofenac sodium and further enhancing adhesiveness of the patch. The acrylic adhesive base is, for example, an adhesive obtained by polymerizing or copolymerizing at least one of (meth)acrylic monomers such as (meth)acrylic acid, 2-ethylhexyl (meth)acrylate, methyl (meth)acrylate, butyl (meth)acrylate, and hydroxyethyl (meth)acrylate. The silicone adhesive base is, for example, mainly composed of a silicone rubber such as polydimethylsiloxane, polymethylvinylsiloxane, or polymethylphenylsiloxane.

The content of an adhesive base in the case of a rubber adhesive base is preferably 10 mass% to 70 mass%, 20 mass% to 50 mass%, 23 mass% to 40 mass%, or 25 mass% to 30 mass% with respect to the total mass of the adhesive layer from the viewpoint of the adhesiveness of the patch. In a case where a mixture of an SIS block copolymer and PIB is used as a rubber adhesive base, the mass ratio of the two is preferably 4:1 to 1:4, 3:1 to 1:1, or 3:1 to 2:1. The content of an adhesive base in the case of an acrylic adhesive base or a silicone adhesive base is preferably 50 mass% to 90 mass% with respect to the total mass of the adhesive layer.

The adhesive layer may further comprise dimethyl sulfoxide (DMSO) for the purpose of dissolving diclofenac sodium to improve skin permeability thereof. The content of DMSO is preferably 1 mass% to 20 mass%, 2 mass% to 10 mass%, 3 mass% to 10 mass%, or 5 mass% to 9 mass% with respect to the total mass of the adhesive layer.

The ratio of diclofenac sodium to DMSO is preferably 1:0.3 to 1:4, 1:0.4 to 1:3, 1:0.6 to 1:3, or 1:0.72 to 1:3 from the viewpoint of improving the skin permeability of diclofenac sodium and the viewpoint of preventing precipitation of crystals of diclofenac sodium.

The adhesive layer may further comprise an organic acid for the purpose of, for example, promoting transdermal absorption of diclofenac sodium or preventing crystals of diclofenac sodium from precipitating over time. Examples of organic acids include aliphatic monocarboxylic acids (such as formic acid, acetic acid, propionic acid, butyric acid, isobutyric acid, valeric acid, caproic acid, enanthic acid, caprylic acid, nonanoic acid, capric acid, lauric acid, oleic acid, linoleic acid, linolenic acid, isostearic acid, sorbic acid, and pyruvic acid), aliphatic dicarboxylic acids (such as oxalic acid, malonic acid, succinic acid, glutaric acid, adipic acid, sebacic acid, maleic acid, fumaric acid, and oxaloacetic acid), aliphatic carboxylic acids such as aliphatic tricarboxylic acids (such as aconitic acid and propanetricarboxylic acid), hydroxy acids (such as glycolic acid, lactic acid, tartronic acid, glyceric acid, hydroxybutyric acid, malic acid, tartaric acid, citric acid, isocitric acid, saccharic acid, gluconic acid, glucuronic acid, ascorbic acid, and erythorbic acid), aromatic carboxylic acids (such as benzoic acid, gallic acid, salicylic acid, acetylsalicylic acid, and phthalic acid), other organic acids (such as mesylic acid and besylic acid), and salts thereof (for example, alkali metal salts such as sodium salts). These organic acids may be used alone or in a combination of two or more thereof. In particular, among these organic acids, citric acid, oleic acid, mesylic acid, and alkali metal salts thereof are preferable and oleic acid is more preferable from the viewpoints of promoting transdermal absorption of diclofenac sodium and preventing crystals of diclofenac sodium from precipitating over time. The content of organic acids is preferably 0.1 mass% to 20 mass%, 0.5 mass% to 10 mass%, 1 mass% to 8 mass%, or 2 mass% to 7 mass% with respect to the total mass of the adhesive layer from the viewpoints of improving the skin permeability of diclofenac sodium and preventing crystals of diclofenac sodium from precipitating over time.

The adhesive layer may further comprise other additives such as a tackifier, a plasticizer, a solubilizer, a stabilizer, and a filler.

Examples of tackifiers include rosin, rosin derivatives such as glycerin ester of rosin, hydrogenated rosin, hydrogenated rosin glycerin ester, and pentaerythritol ester of rosin, alicyclic saturated hydrocarbon resins such as Alcon PI00 (the name of a product manufactured by Arakawa Chemical Industries, Ltd.), aliphatic hydrocarbon resins such as Quintone B170 (the name of a product manufactured by Zeon Corporation), terpene resins such as Clearon P-125 (the name of a product manufactured by YASUHARA CHEMICAL CO., LTD.), and maleic acid resin. Among these, hydrogenated rosin glycerin esters, alicyclic saturated hydrocarbon resins, aliphatic hydrocarbon resins, or terpene resins are preferable and alicyclic hydrocarbon resins are more preferable. Two or more kinds of these tackifier resins may be combined. Incorporation of a tackifier resin can improve the adhesiveness of the adhesive layer and stably maintain other properties. It is preferable that the content of a tackifier resin be, for example, 5 mass% to 60 mass%, 10 mass% to 50 mass%, 30 mass% to 45 mass%, or 35 mass% to 40 mass% based on the total mass of the adhesive layer. A combination of an alicyclic saturated hydrocarbon resin and a hydrogenated rosin glycerin ester is more preferable as a tackifier. The mass ratio of an alicyclic saturated hydrocarbon resin to a hydrogenated rosin glycerin ester is preferably 4:1 to 1:4, 4:1 to 2:3, or 3:1 to 2:1.

Examples of plasticizers include petroleum oils such as paraffinic process oils, naphthenic process oils, and aromatic process oils; squalane; squalene; vegetable oils such as olive oil, camellia oil, castor oil, tall oil, and peanut oil; silicon oils; dibasic acid esters such as dibutylphthalate and dioctylphthalate; liquid rubber such as polybutene and liquid isoprene rubber; liquid fatty acid esters such as isopropyl myristate, hexyl laurate, diethyl sebacate, and diisopropyl sebacate; diethylene glycol; polyethylene glycol; glycol salicylate; propylene glycol; dipropylene glycol; triacetin; triethyl citrate; and crotamiton. Among these, liquid paraffin, liquid polybutene, isopropyl myristate, diethyl sebacate, and hexyl laurate are preferable, liquid polybutene, isopropyl myristate, and liquid paraffin are more preferable, and liquid paraffin is particularly preferable. Two or more kinds of these plasticizers may be combined. The content of a plasticizer is preferably 7 mass% to 70 mass%, 8 mass% to 40 mass%, 10 mass% to 20 mass%, or 12 mass% to 15 mass% with respect to the total mass of the adhesive layer.

Examples of solubilizers include liquid fatty acid esters (such as isopropyl myristate, hexyl laurate, diethyl sebacate, and diisopropyl sebacate), diethylene glycol, triethylene glycol, polyethylene glycol, propylene glycol, dipropylene glycol, triacetin, triethyl citrate, and crotamiton. Among these, diethylene glycol, triethylene glycol, polyethylene glycol, propylene glycol, and dipropylene glycol are preferable, and polyethylene glycol is more preferable. In a case where a solubilizer is polyethylene glycol, the number average molecular weight of polyethylene glycol may be 200 to 20,000, preferably 400 to 6,000, and more preferably 1,000 to 6,000. Two or more kinds of these solubilizers may be combined. The content of a solubilizer is, for example, 0.1 mass% to 10 mass% or 0.5 mass% to 5 mass% based on the total mass of the adhesive layer.

Examples of stabilizers include fatty acid metal salts (such as magnesium stearate), antioxidants (such as tocopherol derivatives, ascorbic acid derivatives, erythorbic acid derivatives, nordihydroguaiaretic acid, gallic acid derivatives, dibutylhydroxytoluene (BHT), butylhydroxyanisole, and 2-mercaptobenzimidazole), and ultraviolet absorbents (such as imidazole derivatives, benzotriazole derivatives, p-aminobenzoic acid derivatives, anthranilic acid derivatives, salicylic acid derivatives, cinnamic acid derivatives, benzophenone derivatives, coumaric acid derivatives, and camphor derivatives). Two or more kinds of these stabilizers may be combined. The content of a stabilizer is preferably 0 mass% to 5 mass% and more preferably 0 mass% to 2 mass% based on the total mass of the adhesive layer. If the adhesive layer comprises a stabilizer, the stability of diclofenac sodium can be further improved.

Examples of fillers include metal oxides (such as zinc oxide and titanium oxide), metal salts (such as calcium carbonate, magnesium carbonate, and zinc stearate), silicic acid compounds (such as kaolin, talc, bentonite, AEROSIL, hydrous silica, aluminum silicate, magnesium silicate, and magnesium aluminometasilicate), and metal hydroxides (such as aluminum hydroxide). Two or more kinds of these fillers may be combined. The content of fillers is preferably 0 mass% to 10 mass% and more preferably 0 mass% to 5 mass% based on the total mass of the adhesive layer.

The adhesive layer may be a single layer consisting of one composition, or may be a plurality of laminated layers having different compositions. The total plaster mass of the adhesive layer is preferably 10 g/m² to 1,000 g/m², more preferably 30 g/m² to 300 g/m², and still more preferably 150 g/m² to 250 g/m² from the viewpoint of making the patch appropriately adhere to the skin.

Next, a backing layer will be described. The backing layer retains an adhesive layer. The backing layer is preferably a monolayer or a laminate of fibers made into the form of fiber (woven fabric, non-woven fabric, or knitted fabric) or a non-porous or porous film (sheet). The material of a backing layer is preferably one or more materials selected from polyesters (such as polyethylene terephthalate (PET), polyethylene isophthalate, polypropylene terephthalate polypropylene isophthalate, polybutylene terephthalate, and polyethylene naphthalate), polyolefins (polymers or copolymers of vinyl monomers such as ethylene, propylene, vinyl acetate, or acrylonitrile), polyamides (nylon or silk), polyurethanes (PU), or cellulose (such as cotton or hemp). The fabric (woven fabric, non-woven fabric, or knitted fabric) may be coated with a rubber composition. The rubber composition comprises a rubber adhesive. The rubber adhesive is, for example, polyisoprene, PIB, polybutadiene, a styrene-butadiene-styrene block copolymer, a SIS block copolymer, styrene-butadiene rubber, styrene-isoprene rubber, or a combination thereof. The rubber composition may comprise a tackifier. The tackifier is, for example, an alicyclic saturated hydrocarbon resin, a hydrogenated rosin ester, a terpene resin, or a combination thereof. In addition, the rubber composition may further comprise additives such as a plasticizer or a filler. The thickness of the backing layer is, for example, 0.1 mm to 2 mm. The basis weight of the backing layer is, for example, 30 g/m² to 200 g/m². In the present specification, the thickness and the basis weight of the backing layer are measured in accordance with the standard of JIS L 1906:2000.

The water vapor transmission rate of the backing layer is preferably greater than or equal to 1,000 g/m²·24 hours or more. When a backing layer having such high water vapor transmission rate is used, DMSO gradually volatilizes from the patch applied to the skin. Therefore, the adhesiveness of the patch is improved and the patch is unlikely to peel off even if it is applied for a long period of time. The upper limit value of the water vapor transmission rate may be 20,000 g/m²·24 hours. If the water vapor transmission rate of the backing layer is within such a range, DMSO more easily volatilizes from the adhesive layer, and therefore the backing layer is more effective for improving the adhesiveness of the patch. The water vapor transmission rate of the backing layer is water vapor transmission rate at 40°C which is defined in accordance with the standard of JIS Z0208:1976 (Testing Methods for Determination of the Water Vapor Transmission Rate of Moisture-Proof Packaging Materials (Dish method)).

When the backing layer is in the form of a fabric, the 50% modulus (JIS L 1018:1999) of the backing layer in both the longitudinal direction (material flow direction) and the transverse direction (material width direction) is preferably 1 N/50 mm to 12 N/50 mm. When the 50% modulus is less than or equal to 12 N/50 mm, the amount of stress applied to the patch due to expansion and contraction of the skin is smaller, and therefore the adhesion to the skin is favorable.

When the backing is a film, it is preferable that the material be highly water vapor transmissible (highly DMSO transmissible), such as polyurethane. Since a film made of a polyurethane has excellent stretchability, it is preferable from the viewpoint of enhancing adhesion of the patch to the skin and stretch followability of the patch.

The backing layer is preferably, for example, a film or non-woven fabric made of a polyurethane, knitted fabric made of polyethylene terephthalate, polyester fabric coated with a rubber composition, or a combination thereof. More specifically, the backing layer is preferably a laminate of a film made of polyurethane and non-woven fabric made of a polyurethane fiber, non-woven fabric made of a PET fiber, or polyester fabric coated with a rubber composition.

In order to secure sufficient adhesive strength and a sufficient skin permeation amount of diclofenac sodium, the application area of the patch is preferably 30 to 300 cm² and more preferably 50 to 150 cm².

The patch can be produced, for example, through the following method, but the present invention is not limited thereto and well-known methods can be used. First, components constituting an adhesive layer are mixed with each other at predetermined proportions to obtain a uniform dissolved material. Next, the dissolved material is spread on a peelable film (a release film or a release liner) or a backing layer to a predetermined thickness to form an adhesive layer. Subsequently, a backing layer is pressed onto the adhesive layer or the peelable film so that the adhesive layer is sandwiched between the peelable film and the backing layer. Finally, the patch can be obtained by cutting the laminate into a predetermined shape. In this case, the release liner is removed at the time of applying the patch. In addition, similarly, the dissolved material may be spread on a backing layer to a predetermined thickness to form an adhesive layer, and then the backing layer may be pressed onto a peelable film (a release film or a release liner).

One patch is applied to the chest, the abdomen, the waist, the back, the upper arm, the thigh, or the like of a human adult once a day and is replaced every day (about 24 hours). The application site is desirably changed every time to avoid skin irritation.

A further embodiment of the present invention will be described below.
[1] A method for relieving cancer pain, the method comprising administering a patch to a patient, wherein the patch comprises a backing layer and an adhesive layer laminated on the backing layer, the adhesive layer comprises an adhesive base and 150 mg to 225 mg of diclofenac sodium, and the patch is applied once a day.
[2] The method according to [1], wherein the patch is a patch comprising at least one selected from the group consisting of a styrene-isoprene-styrene block copolymer and polyisobutylene as the adhesive base.
[3] The method according to [1], wherein the patch is a patch comprising a styrene-isoprene-styrene block copolymer and polyisobutylene as the adhesive base.
[4] The method according to any one of [1] to [3], wherein the patch is a patch wherein the adhesive layer comprises dimethyl sulfoxide.
[5] The method according to any one of [1] to [4], wherein the patch is a patch wherein the adhesive layer comprises an organic acid.
[6] The method according to [5], wherein the patch is a patch comprising oleic acid as the organic acid.
[7] The method according to any one of [1] to [6], wherein the patch is a patch wherein the adhesive layer comprises a tackifier.
[8] The method according to [7], wherein the patch is a patch comprising an alicyclic saturated hydrocarbon resin and a hydrogenated rosin glycerin ester as the tackifier.
[9] The method according to any one of [1] to [8], wherein the patch is a patch wherein the adhesive layer comprises a plasticizer.
[10] The method according to [9], wherein the patch is a patch comprising liquid paraffin as the plasticizer.
[11] The method according to any one of [1] to [10], wherein the patch is a patch having polyethylene terephthalate knitted fabric having a water vapor transmission rate of 1,000 g/m²·24 hours or more, as the backing.
[12] The method according to [1] to [11], wherein the patch is a patch having an application area of 50 to 150 cm².
[13] A patch for use in method for relieving cancer pain, the patch comprising a backing layer and an adhesive layer laminated on the backing layer, wherein the adhesive layer comprises an adhesive base and 150 mg to 225 mg of diclofenac sodium, and the patch is applied once a day.
[14] The patch for use according to [13], wherein the patch is a patch comprising at least one selected from the group consisting of a styrene-isoprene-styrene block copolymer and polyisobutylene as the adhesive base.
[15] The patch for use according to [13], wherein the patch is a patch comprising a styrene-isoprene-styrene block copolymer and polyisobutylene as the adhesive base.
[16] The patch for use according to any one of [13] to [15], wherein the patch is a patch wherein the adhesive layer comprises dimethyl sulfoxide.
[17] The patch for use according to any one of [13] to [16], wherein the patch is a patch wherein the adhesive layer comprises an organic acid.
[18] The patch for use according to [17], wherein the patch is a patch comprising oleic acid as the organic acid.
[19] The patch for use according to any one of [13] to [18], wherein the patch is a patch wherein the adhesive layer comprises a tackifier.
[20] The patch for use according to [19], wherein the patch is a patch comprising an alicyclic saturated hydrocarbon resin and a hydrogenated rosin glycerin ester as the tackifier.
[21] The patch for use according to any one of [13] to [20], wherein the patch is a patch wherein the adhesive layer comprises a plasticizer.
[22] The patch for use according to [21], wherein the patch is a patch comprising liquid paraffin as the plasticizer.
[23] The patch for use according to any one of [13] to [22], wherein the patch is a patch having polyethylene terephthalate knitted fabric having a water vapor transmission rate of 1,000 g/m²·24 hours or more, as the backing.
[24] The patch for use according to [13] to [23], wherein the patch is a patch having an application area of 50 to 150 cm².

### Examples

### (Preparation 1 of Patch Comprising Diclofenac Sodium)

A patch comprising diclofenac sodium was prepared through the following method. Components (showing the composition in the following table) of an adhesive layer were mixed with each other and were spread on a release liner (a PET film subjected to a release treatment) so that the plaster mass was 214 g/m². A backing layer was laminated on the spread adhesive layer to obtain a patch. The patch was cut into a size of 7 cm × 10 cm. PET knitted fabric having a water vapor transmission rate of 5,667 g/m²·24 hours was used as the backing layer.

**[Table 1]**

| Component | Example 1 |
|---|---|
| Diclofenac sodium | 5 |
| Rubber-based adhesive base (SIS/polyisobutylene=19/8) | 27 |
| Tackifier resin (alicyclic saturated hydrocarbon resin/hydrogenated rosin glycerin ester=27/10) | 37 |
| Liquid paraffin | 13 |
| Oleic acid | 5 |
| DMSO | 7 |
| Others | 6 |
| Total (%) | 100 |

### (Preparation of Placebo Patch)

A placebo patch having the same composition except that it does not have diclofenac sodium was prepared through the same method as described above.

### (Evaluation 1 of Analgesic Effect)

During a dose adjustment period, a patch comprising diclofenac sodium was applied to the chest, the abdomen, the waist, the back, the upper arm, the thigh, or the like of patients with painful cancer once a day and was replaced every day. In a case where the analgesic effect was not sufficient, the number of patches to be applied was increased to two and three in this order. The evaluation period was 6 days to 22 days. Only patients who met all of the criteria shown below during this period entered a subsequent double-blind test period.
(1) An average value of visual analogue scale (VAS) values for 3 days immediately before a shift determination day was improved by 15 mm or more as compared to VAS values at a point in time of pre-examination (before the start of the dose adjustment period). The VAS values were measured before bedtime.
(2) Remedial (rescue) actions were not taken in the last 3 days including the shift determination day.
(3) The evaluation results of the degree of improvement in pain of the patients on the shift determination day were "complete improvement," "significant improvement," or "moderate improvement." The degree of improvement in pain was evaluated on a 6-point scale of "complete improvement," "significant improvement," "moderate improvement," "mild improvement," "unchanged," and "exacerbated" based on interviews by medical doctors.

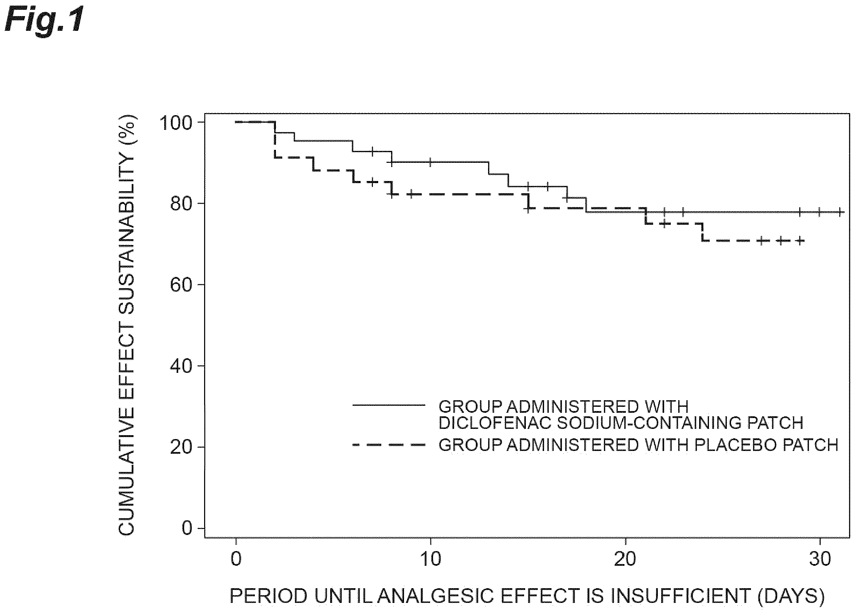

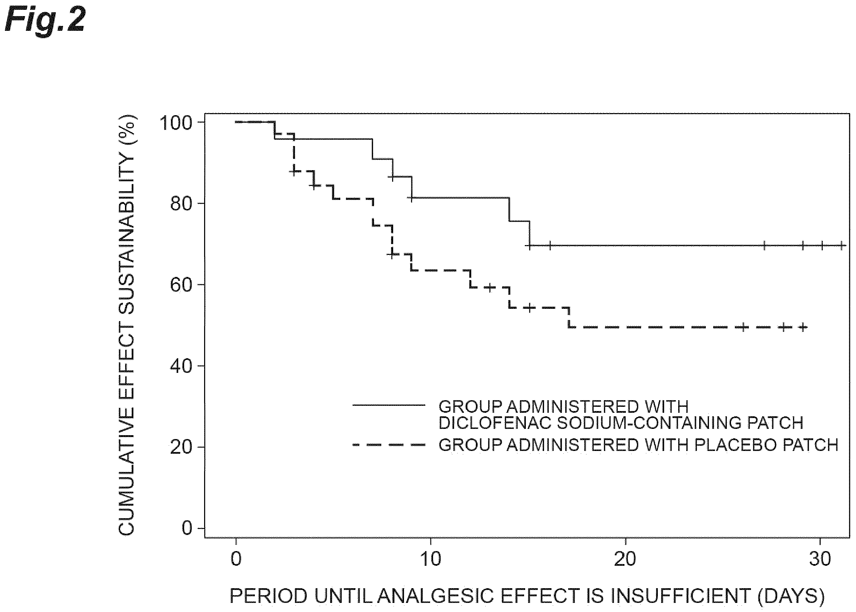

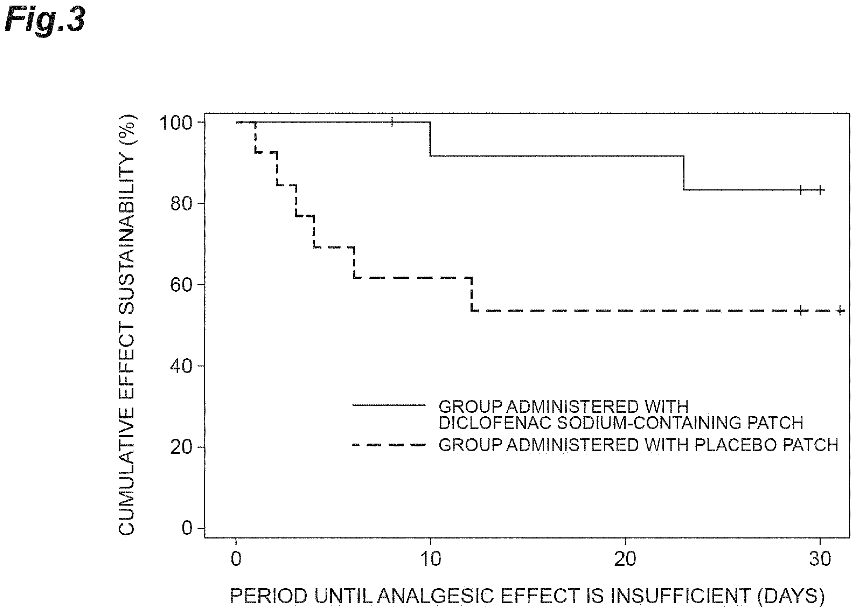

Next, the patients were divided into a diclofenac sodium-comprising patch-administered group (76 cases) and a placebo patch-administered group (79 cases), and the analgesic effect was evaluated through double-blind tests. The dosage of diclofenac sodium was 75 mg (40 cases) (34 cases for placebo), 150 mg (22 cases) (32 cases for placebo), or 225 mg (14 cases) (13 cases for placebo). Graphs in which the period until the patients request discontinuation of the administration due to an insufficient analgesic effect is shown by the Kaplan-Meier curve as a cumulative effect sustainability (%) are shown in Figs. 1 to 3. Fig. 1 shows the Kaplan-Meier curve of the 75 mg administration group, Fig. 2 shows the Kaplan-Meier curve of the 150 mg administration group, and Fig. 3 shows the Kaplan-Meier curve of the 225 mg administration group. The frequency of the requests from patients to discontinue the administration was 8/40 in the 75 mg administration group and 9/34 in the placebo group (log-rank test, p=0.5034), 6/22 in the 150 mg administration group and 14/32 in the placebo group (log-rank test, p=0.1325), and 2/14 in the 225 mg administration group and 6/13 in the placebo group (log-rank test, p=0.0625). As can be seen from these results, the analgesic effect was insufficient when the dosage of diclofenac sodium was 75 mg, whereas the analgesic effect was sufficient when the dosage of diclofenac sodium was 150 mg or 225 mg.

### (Evaluation 2 of Analgesic Effect)

During a dose adjustment period, two patches comprising diclofenac sodium were applied to the chest, the abdomen, the waist, the back, the upper arm, the thigh, or the like of patients with painful cancer once a day and were replaced every day. In a case where the analgesic effect was not sufficient, the number of patches was increased to three. The evaluation period was 2 weeks to 4 weeks. Only patients who met all of the criteria shown below during this period entered a subsequent double-blind test period.
(1) VAS values for 3 days immediately before a shift determination day were improved by 15 mm or more as compared to VAS values at a point in time of pre-examination.
(2) The evaluation results of the degree of improvement in pain of the patients on the shift determination day were "complete improvement," "significant improvement," or "moderate improvement."
(3) The dose of an investigational drug for a dose adjustment period was not changed for 10 days before shift determination.

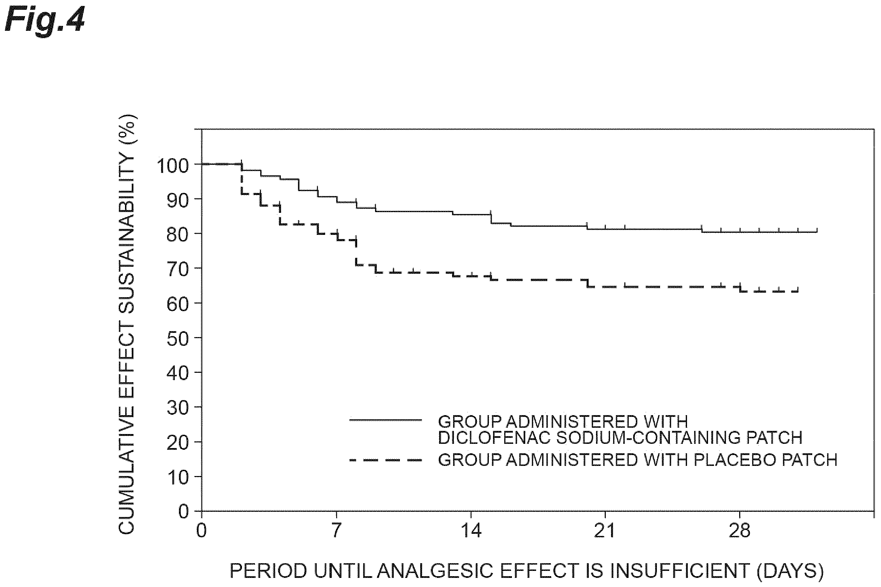

Next, the patients were divided into a diclofenac sodium-comprising patch-administered group (120 cases) and a placebo patch-administered group (118 cases), and the analgesic effect was evaluated through double-blind tests. The dosage of diclofenac sodium was 150 mg (55 cases) (53 cases for placebo) or 225 mg (65 cases) (65 cases for placebo). A case where the VAS values at a point in time when the patients have decided to request discontinuation of the administration due to an insufficient analgesic effect were decreased by more than 15 mm was considered to have an insufficient analgesic effect. A graph in which the period until the analgesic effect is insufficient is shown by the Kaplan-Meier curve as a cumulative effect sustainability (%) is shown in Fig. 4. As can be seen from the results shown in Fig. 4, the analgesic effect was sufficient when the dosage of diclofenac sodium was 150 mg or 225 mg.

### (Pharmacokinetics During Single Dose)

Two patches (application area: 70 cm²) prepared in accordance with the description of Table 1 were applied to the back of each of 14 healthy adult males and peeled off after 24 hours. Blood was collected from immediately before the application to 36 hours after the start of the application, and the drug concentration in blood was measured. The maximum drug concentration in blood (Cmax) and the time (Tₘₐₓ) thereof were determined from the drug concentrations in blood obtained at each time point. In addition, a graph was created with time on the horizontal axis and the drug concentration in blood on the vertical axis, and the area under the curve (AUC) and a half-life of the drug were obtained.

The results are shown in Table 2.

**[Table 2]**

| | Cₘₐₓ (ng/mL) | tmax (hr) | AUC_{0- τ} (ng·hr/mL) | AUC_{0- 24} (ng·hr/mL) | AUC_{0- ∞} (ng·hr/mL) | t_{1/2} (hr) |
|---|---|---|---|---|---|---|
| Number of cases | 14 | 14 | 14 | 14 | 14 | 14 |
| Average value | 55.7 | 13.6 | 1030 | 915 | 1030 | 2.85 |
| Standard deviation | 18.9 | 3.9 | 388 | 367 | 389 | 0.277 |

### (Pharmacokinetics During Repeated Doses)

One patch (application area: 70 cm²) prepared in accordance with the description of Table 1 was applied to the back of each of 14 healthy adult males, peeled off every 24 hours, and replaced with a new patch. This operation was repeated 14 times, and the patch was peeled off on day 15. Blood was collected from immediately before the application to 360 hours after the start of the application, and the drug concentration in blood was measured. The maximum drug concentration in blood (Cₘₐₓ) and the time (Tₘₐₓ) thereof were determined from the drug concentrations in blood obtained at each point in time. In addition, a graph was created with time on the horizontal axis and the drug concentration in blood on the vertical axis, and the area under the curve (AUC) and a half-life of the drug were obtained.

The results are shown in Table 3.

**[Table 3]**

| | Cmax (ng/mL) | | | tmax (hr) | | |
|---|---|---|---|---|---|---|
| | 1st | 7th | 14th | 1st | 7th | 14th |
| Number of cases | 14 | 13 | 13 | 14 | 13 | 13 |
| Average value | 22.9 | 44.1 | 64.0 | 12.9 | 10 | 6.2 |
| Standard deviation | 7.07 | 10.0 | 21.4 | 4.1 | 4.2 | 2.5 |

| | AUC_{0- 24} (ng·hr/mL) | | | t_{1/2} (hr) | | |
|---|---|---|---|---|---|---|
| | 1st | 7th | 14th | After final peeling | | |
| Number of cases | 14 | 13 | 13 | 13 | | |
| Average value | 372 | 813 | 1070 | 2.86 | | |
| Standard deviation | 126 | 169 | 299 | 1.44 | | |

## Claims

1. A patch for relieving cancer pain, the patch comprising a backing layer and an adhesive layer laminated on the backing layer, wherein
the adhesive layer comprises an adhesive base and 150 mg to 225 mg of diclofenac sodium, and
the patch is used by applying the patch once a day.

2. The patch according to claim 1, wherein the adhesive base comprises at least one selected from the group consisting of a styrene-isoprene-styrene block copolymer and polyisobutylene.

3. The patch according to claim 1, wherein the adhesive base comprises a styrene-isoprene-styrene block copolymer and polyisobutylene.

4. The patch according to any one of claims 1 to 3, wherein the adhesive layer comprises dimethyl sulfoxide.

5. The patch according to any one of claims 1 to 4, wherein the adhesive layer comprises an organic acid.

6. The patch according to claim 5, wherein the organic acid is oleic acid.

7. The patch according to any one of claims 1 to 6, wherein the adhesive layer comprises a tackifier.

8. The patch according to claim 7, wherein the tackifier is an alicyclic saturated hydrocarbon resin and a hydrogenated rosin glycerin ester.

9. The patch according to any one of claims 1 to 8, wherein the adhesive layer comprises a plasticizer.

10. The patch according to claim 9, wherein the plasticizer is liquid paraffin.

11. The patch according to any one of claims 1 to 10, wherein the backing is polyethylene terephthalate knitted fabric having a water vapor transmission rate of 1,000 g/m²·24 hours or more.

12. The patch according to any one of claims 1 to 11, wherein an application area is 50 to 150 cm².
